Europäisches Patentamt

European Patent Office

Office européen des brevets

(11) Publication number: **0 315 110 B1**

(12) **EUROPEAN PATENT SPECIFICATION**

(45) Date of publication of patent specification: **28.04.93** (51) Int. Cl.5: **A61K 39/39**

(21) Application number: **88118155.6**

(22) Date of filing: **31.10.88**

(54) **Use of the tumor necrosis factor as adjuvant of the immune response.**

(30) Priority: **02.11.87 IT 2248687**

(43) Date of publication of application:
**10.05.89 Bulletin 89/19**

(45) Publication of the grant of the patent:
**28.04.93 Bulletin 93/17**

(84) Designated Contracting States:
**AT BE CH DE ES FR GB GR LI LU NL SE**

(56) References cited:

JOURNAL OF IMMUNOLOGY, vol. 139, no. 11,
01 December 1987, Am. Ass. Imm. (US);
P.GHIARA et al., pp. 3676-3679*

JOURNAL OF IMMUNOLOGY, vol. 138, no. 6,
15 March 1987, Am. Ass. Imm. (US);
P.SCHEURICH et al., pp. 1786-1790*

JOURNAL OF IMMUNOLOGY, vol. 139, no. 6,
15 September 1987, Am. Ass. Imm. (US);
J.C.LEE et al., pp. 1935-1938*

NATURE, vol. 323, no. 6083, 05-10 September
1986, Neptune, NJ (US); R.PHILIP et al., pp.
86-89*

(73) Proprietor: **SCLAVO S.p.A.**
**Via Fiorentina 1**
**I-53100 Siena(IT)**

(72) Inventor: **Ghiara, Paolo**
**Via Maitani, 5**
**I-53100 Siena(IT)**
Inventor: **Villa, Luigi**
**Via G. Borsi, 53**
**I-50124 Firenze(IT)**
Inventor: **Nencioni, Luciano**
**Via S. Caterina, 13**
**I-53036 Poggibonsi (Siena)(IT)**
Inventor: **Tagliabue, Aldo**
**Chiesa S. Leonardo a Catignano**
**I-53010 Pianella-Castelnuovo Berardenga(IT)**
Inventor: **Boraschi, Diana**
**Chiesa S. Leonardo a Catignano**
**I-53010 Pianella-Castelnuovo Berardenga(IT)**

(74) Representative: **Gervasi, Gemma, Dr. et al**
**Studio Brevetti e Marchi NOTARBARTOLO &**
**GERVASI S.r.l. 33, Viale Bianca Maria**
**I-20122 Milano (IT)**

EP 0 315 110 B1

**Description**

The present invention relates to a new use of the Tumor Necrosis Factor (TNF) in the therapeutic field.

In particular, the present invention relates to the use of TNF for potentiating in a living organism the primary and secondary immune response against natural or synthetic antigenes per se insufficiently immunogenic.

The present invention also relates to a composition containing a pharmacologically effective quantity of said factor.

An antigen is defined as any extraneous substance to a living organism which, once in contact with the immune system, activates a complex series of cellular interactions the scope of which is that of eliminating the same antigen and or reestablishing the preceding equilibrium.

Characteristic aspects of an antigen are: immunogenicity, that is to say the capacity of inducing specific antibodies and the antigenicity, that is to say the capacity of being selectively recognized by the antibodies that the antigen has induced.

As it is known the stimulation of the immune response may be deliberately obtained administering a specified antigen by means of a vaccine.

This procedure permits to obtain a state of immune "memory" in the organism that shall allow a quicker and more effective response thereof in case of a subsequent contact with the antigen itself.

Some antigens, however, are provided with a low immunogenicity and induce an immune response which is not sufficient to impart to the organism an effective protection.

The immunogenicity of an antigen may be enhanced by administering it together with substances, called adjuvants, which potentiate the immune response against the antigen by acting either directly on the immune system or modifying the pharmacokinetic characteristics of the antigen, increasing in this way the interaction time with the immune system.

The more common adjuvants are the Freund adjuvant, emulsion comprising mineral oil and killed bacteria, and aluminium hydroxide.

Said adjuvants are able either to prolonge the antigen residence time in the organism or to produce an aspecific activation of the immune system.

The presence of indesirable effects such as: uprising of a sharp pain and abscesses, in the case of the Freund adjuvant or ineffectiveness towards specific antigens, in the case of aluminium hydroxide, however, make the use thereof in human therapy not completely satisfactory.

It derives from that the necessity of having substances capable of potentiating in vivo the immune response against specific antigen lacking of the drawbacks ot the prior art.

It is also know that the TNF can act as immunomodulator (see P.SCHEURICH et al., Journal of Immunology, 138, no. 6, 3676-3679, (1987); J.C.LEE et al., Journal of Immunology, 139, no. 6, 1935-1938 (1987); R.PHILIP et al., Nature, 323, no.6083, 86-89 (1986)) but none of the cited articles discloses that TNF stimulates in vivo, at doses below those necessary for obtaining antitumor effect, the immune response against specific antigens. specific antigens lacking of the drawbacks of the prior art.

According to that, it has been found that the Tumor Necrosis Factor (TNF), a protein provided with antitumor and anti-carcinemia activity, posseses the property of potentiating in vivo the primary and secondary immune response against specific antigens.

Furthermore, it has been observed that said factor is capable of exerting this activity at doses well below those which have been observed to cause carcinemia shock and damages to the cellular tissue.

One scope of the present invention is therefore the use of the Tumor Necrosis Factor for potentiating in vivo the primary and secondary immune response against natural or synthetic antigens having low immunogenicity. A further object of the present invention is a composition useful in human therapy containing a pharmacologically effective quantity of said factor.

Further objects of the present invention will be apparent from the reading of the following description and examples.

The TNF is a protein produced and released in the outer environment by cells of the monocite - macrophage type as a consequence of activation caused by a bacterial lipopolysaccharide.

Said protein, the aminoacidic sequence of which was deduced from the nucleotidic sequence of cDNA (Pennica D. et a. (1984) Nature, 312: 724), possesses antitumor activity and causes either in vitro or in vivo the necrosis of solid tumors (sarcoma Meth A). Besides these properties, the TNF is able to induce in vivo a cachexic state, that is to say, a metabolic decay, called cachexie, which is often observed during a desease of neoplastic or contageous origin (Beutler, B. and Cerami, A. (1986), Nature, 320:584).

It was not known up to now any immunostimulating property of said factor, least of all its capacity of potentiating in vivo the antibody response against specific antigens in doses well below those necessary for

2

obtaining an antitumor effect (40-200 $\mu$g/kg) or for inducing a cachexic state (1-2 mg/kg), properties which are indesirable for a substance used as adjuvant in human therapy.

According to the present invention, the immune stimulating activity of the TNF was determined by means of the hemolysis plaques method, described by Cunningham and Szeberg (Immunology 14: 599, 1968), method that allows to determine the number of the lymphocytes of the IgM class, characteristic of a primary immune response and antibody of the IgG class, characteristic of a secondary immune response.

In general, said method consists of immunizing mice with the antigen to test, contacting in liquid phase the lymphocytes obtained from the spleen of said mice with the immunizing antigen, adding to said suspension the complement and finally determining, by means of an optical monitor, the number of the lysis zones (Plaque Forming Cells, PFC), formed by the lymphocytes as a consequence of the antigene-antibody reaction. The lysis plaques formed as a consequence of a primary immune response are called direct, whereas those formed as a consequence of a secondary immune respouse are called total.

According to that, and in accordance with the present invention, the immunostimulating activity of the TNF was tested at different dose levels determining g the increase in the number of the cells secreting antibodies of the IgM class and of the IgG class against antigenes with low immunogenicity.

In particular and to the end of better defining the invention without however limiting it, thymus-dependent antigens, such as ram blood corpuscles (SRBC) were used.

According to the present invention male inbred C3H/HeNCr1BR mice and male and female C3H/HeJ mice of 10-12 weeks of age were used, with the same results, and TNF prepared by means of recombinant DNA technics, having purity higher than 95% and an endotoxin content below 0.06 ng/mg of protein, was employed.

The mice were immunized intravenously using an apyrogenic physiologic serum containing the SRBC antigen and, after about 2 hours, some mice were inoculated intraperitoneally with the physiologic serum alone (control), others with a physiologic serum containing TNF at a concentration of from 0.013 picograms (pg) to 8 $\mu$g per kg of body weight and some others with a physiological serum containing the human interleukin-1$\beta$ adjuvant (positive control).

Four days after the immunization, the mice were sacrificed and their spleens withdrawn and mechanically disrupted to separate the lymphocytes secreting anti-SRBC IgM antibodies.

Said lymphocytes, appropriately diluted in a buffer or minimum medium (MEM), were tested with the hemolysis plaques method to determine the immunostimulating effects of TNF on the primary immune response.

The results obtained, reported in the example 1, show that the TNF potentiates the primary immune response against said antigen, that said activity depends on the concentration of the TNF employed and that the minimum effective dose of TNF is of 13 ng/kg.

The possibility that the endotoxin present in the protein could be responsible for its immunostimulating activity was to be excluded, either because the maximum endotoxin content in the inoculated serum was much lower of the minimum active dose (Neter, E. (1969), Current Topics in Microbiology and Immunology, Vol. 47, W. Arber, et al. Springer, New York p. 82), or because the TNF showed its immunostimulating activity in C3H/HeJ mice insensible to the endotoxin (Table 3).

Furthermore, tests carried out employing TNF previously treated at 100 C° for about 20 minutes and TNF treated with anti-TNF rabbit serum showed the absence of immunostimulating activity (Table 4).

The capacity of TNF of potentiating the secondary immune response against SRBC was tested, according to the present invention, by intravenously administering to the mice, 10 days after the first inoculation, the SRBC antigen and immediately afterwards by intraperitoneally administering serums containing different concentrations of TNF.

Also in this casewere observed an increase in the number of cells secreting in each spleen IgG antibodies depending on the concentration employed, and a minimum effective dose of 1.3 ng/kg.

According to the present invention and to the scope of studying the effect of the TNF administration ways on its immunostimulating activity, the mice immunized with SRBC were endovenously inoculated with serums containing different concentrations of TNF, and 4 days after, the increase of the spleen-directed plaques was determined.

The results obtained showed that the minimum effective dose in this case is of 2 pg/kg, that is to say about 6500 times lower of the minimum effective dose observed introducing intraperitoneally the TNF (13 ng/kg).

These results showed that said factor is particularly suitable for potentiating in vivo the primary and secondary immune response against antigens having a low immunogenicity.

According to the present invention, it is possible to use the TNF obtained from cells culture or from engeneered microorganisms or from mammalian tissue extracts, including human beings, in a natural form or in the form of a pharmaceutical acceptable salt thereof.

Said protein may be used as solid susceptible of restoration or as solution or suspension and may be introduced in an organism intraperitoneally, intramuscularly or intravenously.

Particularly suitable compositions to achieve the objects of the present invention are those comprising a pharmaceutical acceptable solvent selected among saline physiologic serum, phosphate buffer and or water and TNF in concentrations of from 0.013 pg/kg to 8 $\mu$g/Kg per dose.

According to the present invention, said compositions may further contain a therapeutically effective quantity of one or more immunizing antigens.

Said antigenes may be selected among natural or synthetic antigens and may be used alone or supported on a macromolecular support.

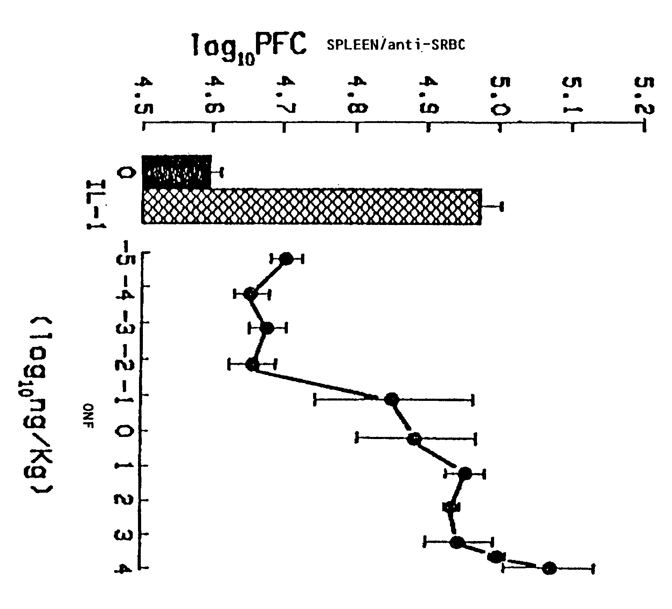

Brief description of Figure 1

The immunostimulating effect of human TNF and of human Interleukin-1$\beta$ on the primary response against the SRBC antigen measured as spleen directed PFC, is reported.

The anti-SRBC directed PFC are determined 4 days after the administration of SRBC in apyrogenic physiologic serum (time 0), of SRBC and human interleukin-1$\beta$ (IL.1 - 20 ng/kg) and of human SRBC and TNF (doses represented in x-coordinate).

Statistical significance:

IL1 against the control 0: p$\leq$0.01
Human TNF against the control 0: doses of from 0.013 pg/kg to 1.3 ng/kg not significant; doses of from 13 ng/kg to 8 ug/kg p$\leq$0.01.

The vertical lines represent the standard deviation of the average values.

The following experimental examples are illustrative and non limiting of the invention.

Example 1

Determination of immunostimulating effect of TNF on the primary response against the SRBC antigen

36 male C3H/HeNCr1Br mice (Calco-Italia) of 10-12 weeks of age and having a weight of about 25 g, are endovenously immunized with 0.2 ml of apyrogenic physiologic serum containing 1-2 x $10^8$ SRBC antigens (SCLAVO, Siena, Italy).

Two hours later the mice are intraperitoneally treated according to the following scheme:
- 3 mice with 0.2 ml of apyrogenic physiologic serum (control):
- 3 mice with 0.2 ml of apyrogenic physiologic serum containing 20 ng/Kg of human Interleukin-1 (IL1$\beta$) (Genzyme Corporation-Boston, MA) having a specific activity of 1x$10^8$ Units per mg of protein;
- 10 groups of 3 mice each with 0.2 ml of apyrogenic physiologic serum containing different doses (from 0.013 pg/kg to 8 $\mu$g/kg) of Tumor Necrosis Factor (TNF) (Biogen Research Corporation, Ginevra, CH) having a specific activity of 1.5 x $10^7$ U/mg of protein and an endotoxin content below 0.06 ng/mg.

Four days later, the mice are sacrificed and the spleen thereof withdrawn and mechanically disrupted to separate the lymphocytes.

The lymphocytes so separated were washed 3 times, using each time 15 ml of minimum medium containing the Earle salts (MEM) (MA. Bioproducts, Walkerville, U.S.A.) and then resuspended in 4 ml of the same washing medium and used to determine the number of cells forming the direct hemolysis plaques (PFC) (Cunningham and Szenber (1968), Immunology 14:599).

In practice, 0.1 ml of each suspension are brought to a final volume of 2.5 ml with MEM medium (final dilution 1:1000) and 100 $\mu$l of each dilution are added, in double, in microplaques holes containing 25 $\mu$l of MEM medium, 25 $\mu$l of a 10% SRBC antigen solution and 25 $\mu$l of guinea-pig serum to a final dilution of 1:64 (complement).

The whole suspensions are immediately transferred, by capillarity, on appropriate superimposed slides.

Said slides, edge-sealed with paraffin are incubated in thermostat at 37° C for 30 minutes and are examined by means of a light contrast monitor, to determine the number of lysis plaques.

The results, reported in Figure 1, show, after the treatment with human IL-1$\beta$ (positive control) and with TNF, an increase in the number of spleen-directed hemolysis plaques.

The latter is also depending on the doses used, the minimum effective dose being of 13 ng/kg.

Example 2

Determination of the immunostimulating activity of TNF on the secondary immune response against SRBC

The mice immunized with SRBC as reported in the preceding example 1, are endovenously inoculated 10 days after the first inoculation with the same dose of SRBC antigen and, immediately afterwards, some mice are intraperitoneally inoculated with 0.2 ml of physiologic serum (control), others with 0.2 ml of physiologic serum containing 20 ng/kg of human IL-1$\beta$ and some other groups width 0.2 ml of physiologic serum containing TNF doses of from 1.3 pg/kg to 1.3 $\mu$g/kg.

Four days after the recall dose, the mice are sacrificed and the total number of spleen-hemolysis plaques (PFC) is determined as described in the example 1 adding, before the complement, a rabbit serum against the mice gamma globulins at a final dilution of 1:80, and to the scope of rendering easier the formation of visible plaques.

The results reported in the following Table 1 show an increase of the total spleen-directed PFC in mice treated with TNF. Said increase is depending on the concentration of the TNF employed and the mininum effective dose is 1.3 ng/kg.

TABLE 1

| Effect of different TNF doses on the secondary response against SRBC | | |
|---|---|---|
| Treatment | Spleen-directed total PFC medium (s.e.m.) | p1 |
| Apyrogenic physiologic serum | 17.378 (15.524-19.453) | |
| IL1 20ng/kg | 33.496 (30.549-36.728) | < .05 |
| TNF.0013ng/kg | 15.596 (13.836-17.579) | n.s. |
| .013ng/kg | 17.179 (13.804-21.579) | n.s. |
| .13ng/kg | 21.528 (18.239-25.410) | n.s. |
| 1.3ng/kg | 29.717 (26.363-33.496) | < .05 |
| 13ng/kg | 39,537 (37.353-34.914) | < .01 |
| 130ng/kg | 30,537 (37.583-41.591) | < .05 |
| 1300ng/kg | 33,884 (26.915-42.658) | n.s. |
| 1) Statistical significance towards the mice group treated with the sole physiological serum | | |

Example 3

Determination of the immunostimulating activity of endovenously inoculated TNF on the primary response against SRBC

36 male C3H/HeNCr1Br mice are endovenously inoculated with a SRBC antigen suspension (100-200 milions/0.2 ml of physiologic serum) and immediately afterwards some mice are inoculated with 0.2 ml of physiologic serum, others with physiologic serum (0.2 ml) containing 20 ug/kg of IL-1$\beta$ and some others with physiologic serum (0.2 ml) containing variable doses of TNF (2,20,200 and 2000 pg/kg).

Four days after the mice are sacrificed and the spleen-directed PFC are determined as reported in the example 1.

The results, listed in table 2, show an increase in the number of spleen/PFC in mice inoculated with different doses of TNF with respect to the control.

TABLE 2

Effect of endovenously inoculated **TNF** on the primary response
against SRBC

| Treatment | Spleen-directed total PFC medium (s.e.m.) | p1 |
|---|---|---|
| Apyrogenic physiologic serum | 47.863 (44.566-51.404) | |
| IL1 20pg/kg | 75.857 (73.857-77.983) | <.01 |
| **TNF** 2 " | 67.143 (62.561-71.945) | <.01 |
| 20 " | 89.125 (79.433-100,000) | <.01 |
| 200 " | 83.176 (74.131-93.325) | <.01 |
| 2000 " | 122.180 (118.577-125.892) | <.01 |

1) Statistical significance towards the control group

Furthermore, it has been observed that, when endovenously administering TNF, the minimum effective dose is 2 pg/kg, that is to say 6500 times lower of the minimum effective dose observed inoculating TNF intraperitoneally (13 ng/kg).

Example 4

Determination of the immunostimulating activity of TNF in mice insensible to the endotoxin

Groups of 3 male C3H/HeJ mice (Calco) of 10 weeks of age, insensible to the endotoxin, are intraperitoneally inoculated with 0.2 ml of apyrogenic physiologic serum containing 100-200 millions of SRBC antigens and, immediately afterwards, some mice are inoculated with only the physiologic serum, others with physiologic serum containing human IL-1$\beta$ (20 ng/kg) and some others with physiologic serum containing 13 ng/kg of TNF.

Four days after the inoculation, the mice are sacrificed and spleen-directed PFC are determined as described in the example 1.

The results, reported in the following table 3 show the absence of any influence of the endotoxin on the immunostimulating activity of TNF.

TABLE 3

| Treatment | Spleen-directed PFC medium (s.e.m.) | p1 |
|---|---|---|
| Apyrogenic physiologic serum | 15.596 (13.677-17.783) | |
| IL1 20ng/kg | 33.343 (33.113-17.783) | $\leq 0.05$ |
| TNF 13ng/kg | 44.978 (40.551-49.888) | $\leq 0.01$ |
| 1) Statistical significance towards the mice group treated with the sole physiologic serum. | | |

Example 5

Effect of temperature and specific antiserum treatment on the adjuvant activity of TNF

A) Groups containing 3 C3H/HeNCr1Br mice are intravenously inoculated with 0.2 ml of physiologic serum containing 100-200 milions of SRBC and, two hours later, 0.2 ml of physiologic serum are intraperitoneally administered to a group, 0.2 ml of physiologic serum containing 130 ng/kg of TNF are administered to a second group, the same quantity of TNF previously treated at 100°C for 20 minutes is administered to a third group.

B) Groups containing 3 mice immunized as in A, are treated with physiologic serum or 13 ng/kg of TNF, or with the same dose of TNF preincubated for 5 hours at 37°C with anti-TNF rabbit serum having a dilution of 1:250 or with the same quantity of normal rabbit serum (NRS).

Four days after inoculation, the spleen-directed PFC are determined as reported in the example 1.

The results, listed in the following table 4, show the absence of any immunostimulating activity of the retreated TNF.

## TABLE 4

| Treatment | Spleen-directed PFC medium (s.e.m.) | p1 |
|---|---|---|
| A Apyrogenic physiologic serum | 47.533 (46.238-48.865) | |
| TNF 130ng/kg | 93.972 (86.298-102.329) | $\leq$ 0.001 |

"

| | | |
|---|---|---|
| 100x20 min. | 46.026 (41.879-50.583) | n.s. |
| B Apyrogenic physiologic serum | 31.117 (28.907-33.496) | |
| TNF 13ng/Kg | 70.795 (66.834-74.989) | ≤ 0.001 |
| "+anti TNF | 29.309 (27.733-30.974) | n.s. |
| " + NRS | 74.131 (67.608-81.283) | ≤ 0.01 |

1)  Statistical significance towards the control group; n.s. = not significant

## Claims

**Claims for the following Contracting States : AT, BE, CH, DE, FR, GB, LI, LU, NL, SE**

1.  Use of Tumor Necrosis Factor for preparing a pharmaceutical composition useful for potentiating in vivo the primary and secondary immune response against natural or synthetic antigen.

2.  A pharmaceutical composition useful for potentiating in vivo the primary and secondary immune response against natural or synthetic antigens comprising a pharmaceutically acceptable solvent and the Tumor Necrosis Factor, wherein said factor has a concentration of from 0.013 pg/kg to 8 $\mu$g/kg.

3.  A composition according to Claim 2, further comprising a therapeutically effective amount of a natural or synthetic antigen.

**Claims for the following Contracting States : ES, GR**

1.  A method for preparing a pharmaceutical composition, useful for potentiating in vivo the primary and secondary immune response against natural or synthetic antigens, wherein : TNF at a concentration of from 0.013 pg/kg to 8 $\mu$g/kg, obtained from cells culture or from engineered microorgaism or from mammaliam tissuue extracts, is mixed with a pharmaceutically acceptable solvent.

2.  Method according to claim 1 wherein the pharmaceutical acceptable solvent is selected from a group consisting of saline physiologic serum, phosphate buffer or water.

3.  Method according to claims 1 and 2, wherein the pharmaceutical composition contains an effective amount of a natural or synthetic antigen.

9

EP 0 315 110 B1

**4.** Method according to claim 3, wherein said antigen is supported on a macromolecular support.

**5.** Method according to claims 3-4, wherein said antigen is thymus-dependent antigen.

**6.** Method according to claim 5, wherein the antigen is SRBC antigen.

**Patentansprüche**
**Patentansprüche für folgende Vertragsstaaten : AT, BE, CH, DE, FR, GB, LI, LU, NL, SE**

**1.** Verwendung eines Tumornecrosfaktors zur Herstellung einer pharmazeutischen Zusammensetzung, nützlich für die in vivo Potenzierung der primären und sekündären Immunantwort gegen natürliches oder synthetisches Antigen.

**2.** Pharmazeutische Zusammensetzung, nützlich zur in vivo Potenzierung der primären und sekundären Immunoantwort gegen natürliche oder synthetische Antigene, umfassend ein pharmazeutisch akzeptables Lösungsmittel und den Tumornecrosfaktor, worin der Faktor eine Konzentration von 0,013 pg/kg bis 8 $\mu$g/kg aufweist.

**3.** Zusammensetzung nach Anspruch 2, dadurch **gekennzeichnet,** daß sie weiterhin eine therapeutisch effektive Menge eines natürlichen oder synthetischen Antigens enthält.

**Patentansprüche für folgende Vertragsstaaten : ES, GR**

**1.** Verfahren zur Herstellung einer pharmazeutischen Zusammensetzung, nützlich für die in vivo Potenzierung der primären und sekundären Immunantwort gegen natürliche oder synthetische Antigene, worin TNF bei einer Konzentration von 0,013 pg/kg bis 8 $\mu$g/kg, erhalten aus einer Zellkultur oder von technischen Mikroorganismen oder von Säugetiergewebeextrakten, mit einem pharmazeutisch aktzeptablen Lösungsmittel gemischt wird.

**2.** Verfahren nach Anspruch 1, dadurch
**gekennzeichnet,** daß das pharmazeutisch akzeptable Lösungsmittel aus einer Gruppe ausgewählt ist, bestehend aus physiologischem Salzserum, Phosphatpuffer oder Wasser.

**3.** Verfahren nach Anspruch 1 und 2, dadurch
**gekennzeichnet,** daß die pharmazeutische Zusammensetzung eine effektive Menge eines natürlichen oder synthetischen Antigens enthält.

**4.** Verfahren nach Anspruch 3, dadurch
**gekennzeichnet,** daß das Antigen auf einem makromolekularen Träger getragen wird.

**5.** Verfahren nach den Ansprüchen 3 und 4, dadurch
**gekennzeichnet,** daß das Antigen thymus-abhängiges Antigen ist.

**6.** Verfahren nach Anspruch 5, dadurch
**gekennzeichnet,** daß das Antigen SRBC-Antigen ist.

**Revendications**
**Revendications pour les Etats contractants suivants : AT, BE, CH, DE, FR, GB, LI, LU, NL, SE**

**1.** Utilisation du facteur de nécrose tumorale pour préparer une composition pharmaceutique utile pour potentialiser in vivo la réponse immunitaire primaire et secondaire contre un antigène naturel ou synthétique.

**2.** Une composition pharmaceutique utile pour potentialiser in vivo la réponse immunitaire primaire et secondaire contre des antigènes naturels ou synthétiques, comprenant un solvant pharmaceutiquement acceptable et le facteur de nécrose tumorale, dans laquelle ledit facteur a une concentration de 0,013 pg/kg à 8 $\mu$g/kg.

10

3.  Une composition selon la revendication 2 comprenant de plus une quantité thérapeutique efficace d'un antigène naturel ou synthétique.

**Revendications pour les Etats contractants suivants : ES, GR**

1.  Un procédé pour préparer une composition pharmaceutique utile pour potentialiser in vivo la réponse immunitaire primaire et secondaire contre des antigènes naturels ou synthétiques, dans lequel on mélange du TNF à une concentration de 0,013 pg/kg à 8 $\mu$g/kg, obtenu à partir de cultures cellulaires ou d'un microorganisme modifié par génie génétique ou à partir d'extraits de tissu de mammifère, avec un solvant pharmaceutiquement acceptable.

2.  Procédé selon la revendication 1, dans lequel le solvant pharmaceutiquement acceptable est choisi dans un groupe constitué du sérum salé physiologique, du tampon phosphate et de l'eau.

3.  Procédé selon les revendications 1 et 2, dans lequel la composition pharmaceutique contient une quantité efficace d'un antigène naturel ou synthétique.

4.  Procédé selon la revendication 3, dans lequel ledit antigène est porté sur un support macromoléculaire.

5.  Procédé selon les revendications 3 et 4, dans lequel ledit antigène est un antigène thymodépendant.

6.  Procédé selon la revendication 5, dans lequel l'antigène est l'antigène hématies de mouton.